# EUROPEAN PATENT APPLICATION

(11) **EP 3 244 252 A1**
(43) Date of publication of application: **15.11.2017**
(21) Application number: 16382211.7
(22) Date of filing: 12.05.2016
(51) Int. Cl.: G02B 27/01, G02C 7/08

(54) **SYSTEM, METHOD AND COMPUTER PROGRAM FOR IMPROVING THE VISION OF PEOPLE WITH A MACULAR DISORDER**

(71) Applicant: Fundacion Tekniker, 20600 Eibar (Guipuzkoa) (ES)
(72) Inventor: OÑATE GUTIERREZ, Roberto, 20600 Eibar (GUIPÚZCOA) (ES); MABE ALVAREZ, Jon, 20600 Eibar (GUIPÚZCOA) (ES); PALOMINO MUÑOZ, Antonio, 20600 Eibar (GUIPÚZCOA) (ES)
(74) Representative: Balder IP Law, S.L.

(57) **Abstract**

A system of assistance for a person with a macular disorder in one eye, wherein the system is integrated in or coupled to a pair of spectacles (5), comprising: a camera (1) to capture an image (13) situated in the field of view (12) of said person and to measure the amount of ambient light; some processing means (6) to process said image (13), wherein said processing means (6) comprise software means for implementing image-processing algorithms so as to obtain a retouched virtual image (22) from said image (13); a display (3) in front of the eye affected by a macular disorder; a projection system (2) to receive said retouched virtual image (22) and to project (32) the retouched image (22) onto said display (3), so that said individual sees said retouched image projected (32) instead of the real image (12) present in the person's field of view; and a sheet (4) of variable opacity disposed next to the display (3), the display (3) being situated between the sheet (4) and the eye (13), the sheet (4) being configured so as to modulate its opacity in accordance with the ambient light and thus improve the contrast of the image projected (32) on the display (3).

## Description

### TECHNICAL FIELD

The present invention belongs to the field of Ophthalmology and Optics and, in particular, to devices to improve vision in people with a macular disorder, such as macular degeneration, macular dystrophies such as Stargardt's disease, traumatic macular disorders, macular disorders due to sunlight, diabetic macular disorders, idiopathic macular holes, etc.

### BACKGROUND OF THE INVENTION

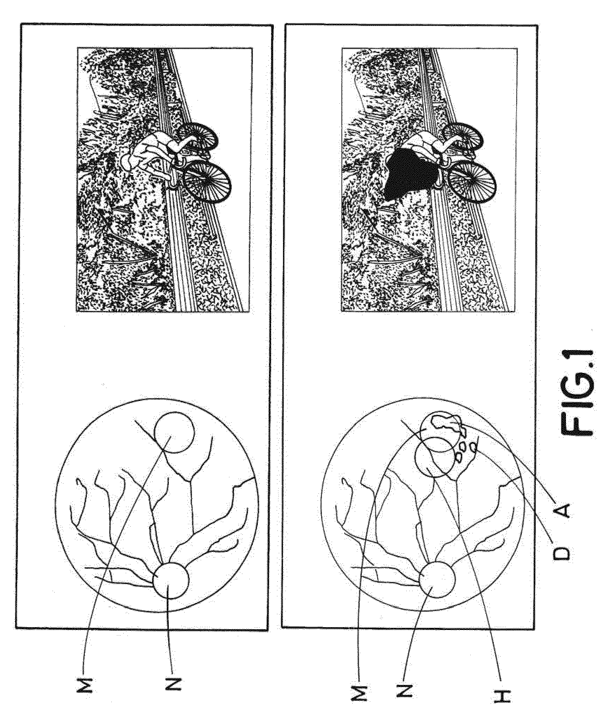

The macula is the central area of the retina and therefore is the part of the retina responsible for central vision; that is to say the part with which we see detail, read and, for example, see people's faces. Macular disorders, which may be caused by a number of diseases, such as macular degeneration, macular dystrophies such as Stargardt's disease, traumatic macular disorders, macular disorders due to sunlight, diabetic macular disorders, idiopathic macular holes, etc., result in deterioration of the macula. In the retina (back of the eye), light-sensitive cells send visual signals to the brain. Clear, rectilinear (central) vision, colour and fine details are processed by the macula. Damage in this area results in blind spots and blurred or distorted vision, that is to say a central scotoma is produced. When the macula has deteriorated, many everyday activities such as driving, identifying faces and reading become increasingly difficult. Macular degeneration is today considered the most common cause of blindness in developed countries and, even though it rarely leads to complete blindness since peripheral vision is spared, it has a serious effect on patients' independence and quality of vision. The incidence of age-related macular degeneration (AMD) is rising owing to the ageing of the population and improvements in the treatment of ophthalmological diseases. It is estimated to affect 10% of people over the age of 65 and this proportion increases with age, as it becomes progressively worse. It may affect just one eye or both eyes, but someone who has macular degeneration in one eye is more likely to develop it in the other eye than someone who does not have the disorder. Figure 1 shows at top left a diagram of a healthy eye, on which the optic nerve N and the macula M have been labelled. To the right of the eye is an example of an image captured by the healthy eye.

At bottom left the same eye is shown suffering from certain macular disorders, such as subretinal haemorrhage H, atrophy A and drusen D. The illustration to the right of this eye shows how these macular pathologies affect the view of the same image shown in the upper part of the figure. As may be appreciated, the patient experiences a loss of central vision.

In addition to medicinal therapy, several techniques have been developed to mitigate the effects of AMD. A notable example is the implant developed by *VisionCare Ophthalmic Technologies,* which consists of a miniature Galilean telescope that is surgically implanted in the eye to replace the physiological lens. The thin telescope lens magnifies images on the retina so that healthy cells can carry out the function of the non-functional cells of the macula. The implant allows patients who have it incorporated in their eye to regain the ability to perform tasks such as reading or watching television. The telescope is implanted in one eye, while the other eye, without an implanted telescope, is used for peripheral vision, which is necessary for activities like walking. Other intraocular mini-telescopes exist (known as IOL VIP, 'Intraocular Lens for Visually Impaired People'), which are less capable of enlarging the image (x1.2) and can be used in both eyes because they do not reduce peripheral vision too much. However, they have not produced outstanding results.

Some disadvantages of this technique are listed here: patients require therapy to get used to their new ocular situation (using one eye for central vision and the other for peripheral vision); the technique is not applicable to all patients with macular degeneration (for example, those who have had cataract surgery with an intraocular lens, since that means there is no room for the mini-telescope). There are also the risks inherent in any eye surgery. Since the mini-telescope is a relatively large device, a large surgical wound has to be made to implant it, resulting in major astigmatism. Moreover, this operation is usually performed on older people who have a small quantity of endothelial cells, which is a frequent cause of corneal decompensation. Lastly, it has to be mentioned that this technique is invasive and practically irreversible.

Alternatively, in the last few years a wide range of image projection solutions has been developed, constituting a family of devices known as Near Eye Displays, Head-Mounted Displays (HMDs) or Virtual Reality Displays, which project a virtual or real image in the field of view of one or both eyes. This projected image is perceived by the human eye and brain as if it were actually at a given distance and relatively much larger than the minute size of the display and the optics mounted in front of the eye. Some examples of documents that demonstrate the operating principles of these technologies are 'DLP® Technology for Near Eye Display', White Paper, Texas Instruments, Literature Number DLPA051, September 2014, and EDN Network, 'An Insider's guide to designing near-eye displays', Carlos Lopez, Texas Instruments, 3 February 2015.

Additionally, European patent EP1779159B1 relates to image projection and describes an optical device based on a light-transmitting substrate that incorporates several reflective surfaces, which can be used in HMD applications. International patent application WO2011/089042A1 also presents a solution for the short-distance projection of large images. United States patent US8135227B2 describes a method and apparatus for augmenting sight in an individual. The apparatus comprises a camera for obtaining an image of what the individual is seeing, a captured image input device, a screen and a processor. All this is carried by the user. The processor modifies the captured image and shows the modified image on the screen so that the sight-impaired user can see it well. However, it has been observed that, when a screen is placed on the lens of the spectacles, the user, who is already suffering from severe sight problems, appears confused by the two images in front of him or her: one being the real image viewable through the lens of the spectacles, and the other the modified image shown on the screen.

### DESCRIPTION OF THE INVENTION

The present invention seeks to resolve the above-mentioned disadvantages by means of a method, system and computer program for improving the sight of people with a macular disorder. The system is implemented on conventional spectacles, thus avoiding the need for surgery.

The operating principle of the proposed system is based on the operation of the human focusing system itself, which tends to focus the image of interest that one wishes to process onto the centre of the macula. That is, the human focusing system attempts to adjust itself until the object of interest projects its image onto the centre of the macula. That means that in patients with macular degeneration it is precisely the object of interest that can be viewed least well, whereas the periphery of the object can still be 'seen' as the object is projected onto healthy cells. This is principally a problem for everyday activities such as reading or recognising faces.

The system of the invention uses artificial means to project an N-times magnified image of the object of interest and improves the contrast of the display on which the image is projected. As a result, a proportionally smaller area of the image of the object remains affected by the loss of central vision, enabling the patient to interpret and recognise said object of interest. Additionally, to mitigate additional effects caused by macular degeneration, the application of colour processing techniques to the images to be projected allow the patient to perceive an improved chromatic sensation.

In a first aspect of the invention, a system of assistance is provided for a person with a macular disorder in at least one eye, wherein the system is integrated in or coupled to a pair of spectacles. The system comprises: a camera configured to capture at least one image situated in the field of view of said person and to measure the amount of ambient light; processing means configured to process said image captured by the camera, wherein said processing means comprise software means for implementing at least one image-processing algorithm configured to obtain a virtual image retouched in accordance with said macular disorder from said image captured by the camera; a display situated in front of the eye affected by a macular disorder; a projection system configured to receive said virtual image retouched in accordance with said macular disorder by the processing means and to project the retouched image onto said display, so that said person with said macular disorder sees said retouched image projected instead of the real image present in the person's field of view; and a sheet of variable opacity disposed next to the display, the display being situated between the sheet of variable opacity and the person's eye, the sheet of variable opacity being configured to modulate its opacity in accordance with the ambient light and thus improve the contrast of the image projected on the display.

In a possible embodiment, the processing means are embedded in the frame of a pair of spectacles.

In a possible embodiment, the camera is situated on the front part of the frame of a pair of spectacles, or in the area in which the temple begins, or on the bridge intended to rest over the nose.

The sheet of variable opacity may be an electrochromic crystal, which changes its opacity through the application of an electric voltage in accordance with the ambient light level detected by the camera, or a photochromic crystal, which changes its opacity in accordance with the ambient light directly.

Preferably, the projection system comprises a plurality of light sources and diffraction means.

Preferably, the display comprises a waveguide system.

In a possible embodiment, the at least one processing algorithm comprises a magnification algorithm configured to enlarge/reduce the captured image N times, N being a real number varying between 0.1 and 10, most preferably between 2 and 4. Optionally there is also implemented a filtering and chromatic compensation algorithm configured to absorb short-wave radiation below 600 nm. Also optionally, there is implemented at least one algorithm for the compensation of refractive errors.

In another embodiment of the invention, an ensemble/assembly is provided for the assistance of an individual with a macular disorder in at least one eye, comprising: a spectacle frame and a system such as that described above installed in said spectacle frame.

In another aspect of the invention, a method of assistance is provided for an person with a macular disorder in at least one eye, comprising: capturing in a camera an image situated in the field of view of said person and measuring the amount of ambient light; processing said image captured by the camera so as to obtain a retouched virtual image of the captured image; projecting said processed image from a projector onto a display situated in front of the eye affected with said macular disorder; improving the contrast of the projected image by means of a sheet of variable opacity disposed next to the display.

In a possible embodiment, the stage of processing said captured image comprises applying at least one image-processing algorithm configured to obtain a retouched virtual image from said image captured by the camera.

In a possible embodiment, the at least one processing algorithm comprises enlarging/reducing the captured image N times, N being a real number varying between 0.1 and 10, most preferably between 2 and 4. Optionally the algorithm also comprises filtering the image and applying chromatic compensation to it, based on absorbing short-wave radiation below 600 nm, and optionally compensating for refractive errors.

In a final aspect of the invention, a computer program is provided that comprises computer programming code instructions for implementing the method described above.

The advantages of the invention will become apparent from the description that follows.

### BRIEF DESCRIPTION OF THE DRAWINGS

To complement the description and with the aim of helping to provide a better understanding of the characteristics of the invention in accordance with an example of a practical embodiment of the invention, the following set of figures is included as an integral part of the description. What is represented in these figures is not restrictive and is for illustrative purposes only.
Figure 1 shows a diagram of a healthy eye (top left) and an example of an image captured by the healthy eye (top right). The figure also shows (bottom left) the same eye affected by some macular disorders and the same image as above, this time captured by the damaged eye (bottom right).
Figures 2A to 2E show several diagrams of systems according to the present invention implemented on a conventional spectacle frame. Figures 2C and 2D represent the electrochromic/photochromic effect darkening the spectacle lens. In figure 2E the processing means can be seen situated on the temple of the spectacles.
Figure 3 shows a diagram of the system of the invention according to a possible configuration, the operation of which is based on a projection system and a display that reflects the video into the eye (see-through type of configuration).

### DESCRIPTION OF AN EMBODIMENT OF THE INVENTION

In this text, the term 'comprises' and its variants (such as 'comprising', etc.) should not be interpreted in an excluding sense, that is, these terms do not exclude the possibility that what is described may include other elements, steps, etc.

In the context of this invention, the term 'approximately' and its family of terms (such as 'approximate', etc.) should be understood as indicative values very close to those accompanying the previously mentioned term. That is to say, a deviation from an exact value should be accepted within acceptable limits, since a person skilled in the art will understand that said deviation from the indicated values is inevitable due to imprecisions of measurement, etc. The same applies to the terms 'around' and 'substantially'.

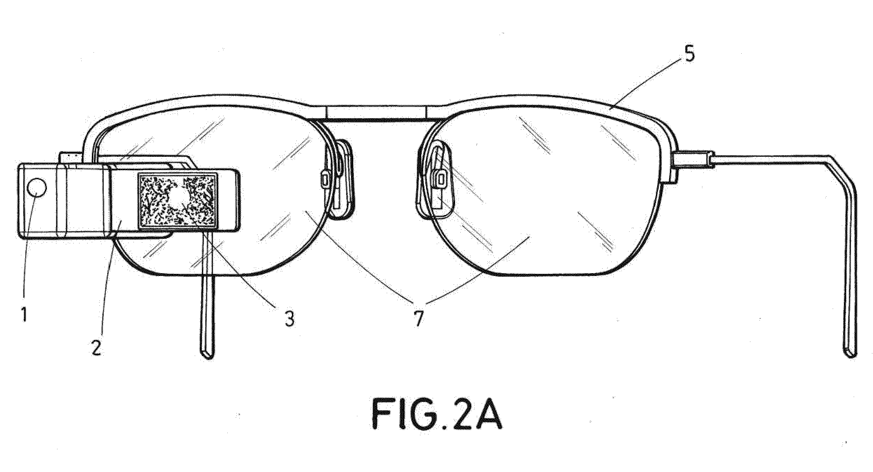

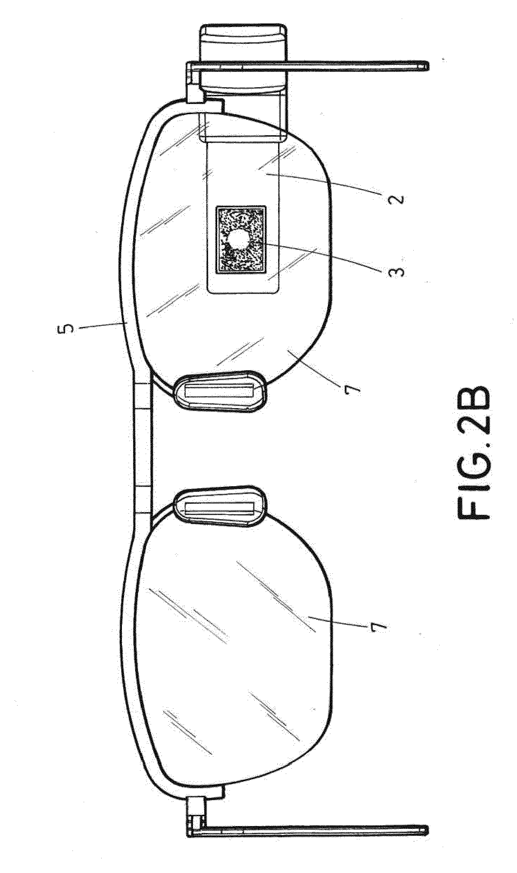

Figures 2A to 2E show different views and implementations of a system of assistance or help for patients with a macular disorder, according to possible embodiments of the invention. A system of assistance according to the invention is formed by several components disposed on a conventional spectacle frame 5 or coupled to another fixing element such as a helmet, headband, etc. Some of the components houses an ensemble/assembly of algorithms implemented by software means and adjustable to the particular lesions of each individual patient. The system may be installed to assist the right eye, as shown in the figures, the left eye (not illustrated) or, through duplication, to assist both eyes (not illustrated). The same system may be used for the left or right eye simply by rotating the device and configuring the orientation of the projection, top to bottom for the left eye and bottom to top for the right eye. That is, the manner of reading the image from the camera is configured (e.g. top to bottom for the left eye and bottom to top for the right eye). This inverse reading of the image results in changing the orientation of the projected image.

The system is implemented by means of an optical projection and illumination system and a display (see-through type of configuration). As shown diagrammatically in figures 2A-2E, the system has a camera 1, preferably a microcamera. The camera 1 is a conventional camera for capturing video or photographs in real time. The camera 1 is totally programmable, that is, it can be configured to record video at the resolution that is selected, within the limits permitted by the camera, or to take photographs, also at the resolution that is selected. Preferably, the camera 1 is configured to record video. The camera 1 makes it possible to record in video form what is in front of the eyes of the person carrying the system. That is, the camera 1 is oriented towards the field of view of the person carrying the system and acts as an 'artificial eye', recording the images that the user has in front of his or her eyes. The camera 1 is located on the frame of the spectacles 5, for example on the front thereof, either in the area where the temple begins (the junction between the left- (or right-)hand frame and the origin of the temple), as shown in figures 2A-2E, or on the bridge intended to rest over the nose, that is, towards the middle of the spectacles (not illustrated). The camera 1 is fixed or assembled on the frame of the spectacles 5 by conventional fixing or assembly means. The camera 1 is also configured to capture or to measure the amount of ambient light. Optionally, the camera may also include a lens with the ability to change focus in order to permit close or distant focusing, with an angle of view.

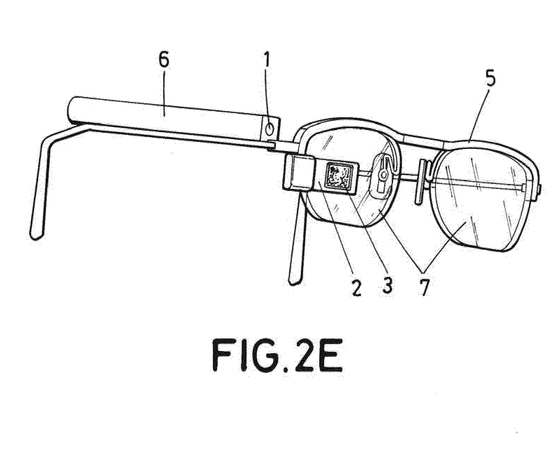

The images captured by the camera 1 are processed in processing means, such as a mini-computer or processor, which may be coupled to the frame 5 of the spectacles, as illustrated in figure 2E, reference 6, or which may be an element separate therefrom, for example carried by the user on clothing, in a pocket, on the wrist, or in any other way. The processing means 6 adapt the images captured by the camera 1 in accordance with the needs of each individual user.

The system also comprises an optical projection and illumination system (or microprojector) 2. By means of the optical projection and illumination system 2, the image (or images) obtained by the camera 1 and processed by the processing means 6 is projected on a display 3 (preferably implemented by means of a waveguide). Preferably the microprojector 2 is coupled to the frame of the spectacles 5, for example in the area where the processing means 6 may be coupled.

The system also comprises a display 3, implemented by means of a waveguide, and preferably a sheet or additional surface 4 of variable opacity. This sheet 4 of variable opacity may be either an electrochromic crystal (also termed electrochromic glass) or a photochromic crystal (also termed photochromic glass). These crystals are conventional and lie outside the scope of the present invention. Preferably the display 3 is coupled to the spectacle lens or is superimposed thereon. The microprojector 2 comprises illumination optics (one or more light sources) and diffraction optics, as well as a video input to receive the signal from the processor. The illumination optics is responsible for casting light towards the display 3 so that the image is correctly generated thereon. The illumination optics may be implemented by means of one or more light sources. The diffraction optics projects the image or images (video) that reach it, in a similar manner to conventional slide projectors. In a possible embodiment, the diffraction optics is implemented by means of micromirrors. The waveguide 3 is the element on which the projected image is seen. With the waveguide 3, the projection is perpendicular and directs the image into the eye. The sheet of variable opacity 4 is an auxiliary element that may be added to increase the contrast of the image on the display in very bright surroundings (in sunshine, for example). This sheet of variable opacity may be implemented for example by means of an electrochromic crystal, which changes its opacity/transparency when an electric voltage is applied to it, said voltage being applied from the spectacle frame in accordance with the ambient light level detected by the camera, or by means of a photochromic crystal, which changes its opacity/transparency in accordance with the ambient light directly.

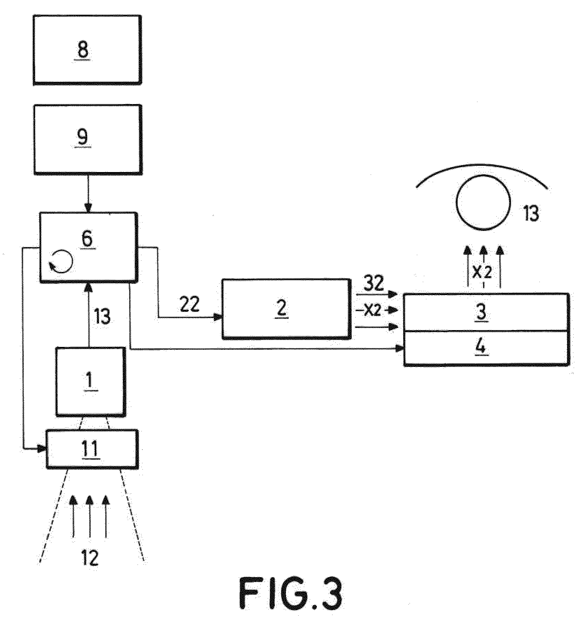

The operation of the system is explained in detail in relation to figure 3. Figure 3 shows a block diagram of the system, implemented with a configuration that operates based on a microprojector and microdisplay (see-through type of configuration), similar to the system installed on the frame of a pair of spectacles illustrated in figures 2A-2E. The microprojector 2 is similar to those used for projection onto a screen. The microprojector 2 is based preferably on the synchronised control of three sources of Red, Green and Blue wavelength light together with a structure of micromirrors (diffraction optics) manufactured with MEMS technology and a dedicated optics. The micromirrors reflect in a controlled and synchronised fashion the light emitted by the light sources generating a photon pattern that represents the image or video to be projected. Conventional examples of microprojectors may be found in Texas Instruments DLP technology or Microvision PicoP projectors. The system described uses a conventional microprojector, which lies outside the scope of the present invention. The projection system 2 projects the images recorded by the camera 1 on the waveguide 3.

A 'see-through' type of display 3 (preferably a microdisplay) is selected. The 'see-through' display 3 allows the patient to continue seeing by him or herself through the spectacle lens 7. Most preferably the display 3 is based on a conventional waveguide system. In an example of an embodiment, a display 3 with the following characteristics is selected:
Display Full Colour Landscape Mode 4:3
Field of View (diagonal) 70" at 4 m
Resolution 33 pixels / degree
Brightness -3000 Cd/m²

In another example of an embodiment, a display 3 with the following characteristics is selected:
Display Full Colour Landscape 16:9
Field of View (diagonal) 61" at 4 m
Resolution 126 pixels / degree
Brightness >3000 Cd/m²

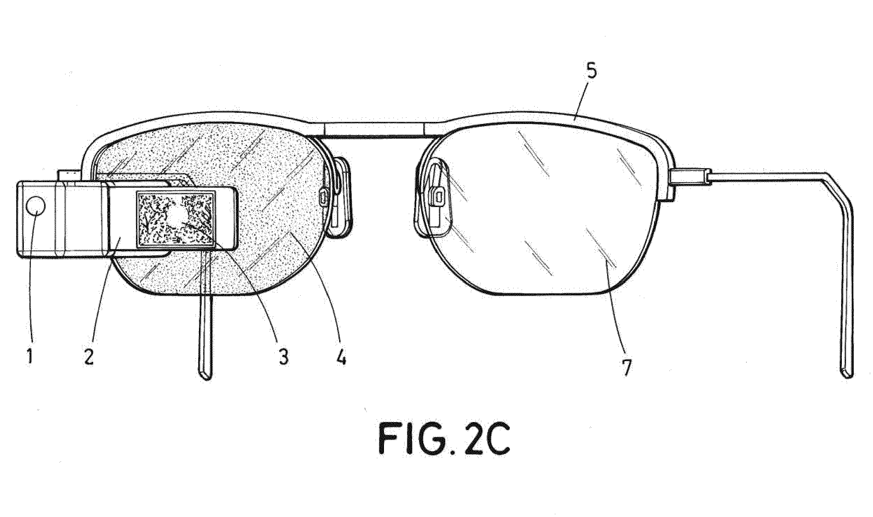

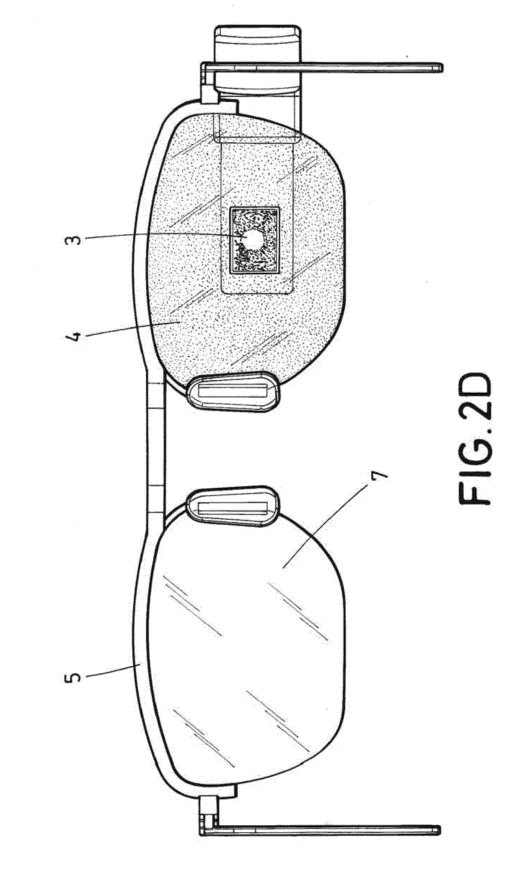

However, it has been observed that the user, who suffers from severe sight problems, often appears confused by the two images that are simultaneously in front of him or her: the real image 12, accessible through the spectacle lens 7, and the modified image 32 shown on the screen or display 3. To mitigate this problem, a sheet or surface of variable opacity 4 has been added to the system, which for example may be an electrochromic crystal or a photochromic crystal, illustrated in figures 2C and 2D and represented schematically in figure 3, to improve the contrast. Resolution is thereby improved. This sheet of variable opacity 4 is added to the display 3 and to the conventional spectacle lens. The sheet of variable opacity 4 may be similar in size to the conventional spectacle lens. For example, in figure 2C, the sheet of variable opacity 4 overlaps the original spectacle lens completely. Or it may be similar in size to the display or waveguide 3. That is, to improve the contrast, a sheet of variable opacity 4 is used behind the waveguide system (display 3). In cases where the sheet of variable opacity 4 is an electrochromic crystal, the opacity of the crystal may be changed by means of an electric current. In this way the system can modulate the opacity of the crystal 4 in accordance with the ambient light and thereby improve the contrast of the projected image.

The camera 1, preferably based on a CMOS sensor, captures input images 12 of what is in front of the user's eyes and spectacles. Camera 1 is preferably supplemented with a focusing system 11 based on a lens and a focusing motor. Preferably the camera 1 records video, so that the user can continuously see what he or she is looking at. The images recorded by the camera 1 pass to the processing means 6, which process them and supply them to the projection system 2. In figure 3, the arrow that goes from the camera 1 to the processing means 6 represents a signal that comprises the 'real' captured video. The processing means 6, which for example may be a CPU, a microprocessor or any other conventional processing means, are situated preferably in a temple of the spectacles or in an external case or capsule fixed to the spectacles, preferably to the temple, as shown in figure 2E. The processing means 6 are connected to the camera 1 by means of a standard camera port, such as a MIPI or parallel port. The processing means 6 are connected to the projection system 2 by means of a standard output-to-screen port, such as HDMI or similar. The processing means 6 process the images and supply them to the projection system through said port. The interface between the projector 2 and the display 3 is an optical interface and lies outside the scope of the invention. The focus applied by the focusing system 11 may be controlled and adjusted from the processing means 6, as shown by the arrow from the processing means 6 to the focusing system 11. The processing means 6 are responsible for handling/managing the other components (camera 1, display (waveguide system) 3 and projector 2) and for the processing and execution of algorithms for providing the user with visual assistance. As has been mentioned, in a particular embodiment, the processing means 6 are embedded in the temple of the spectacles 5. To the spectacles (preferably to the temple of the spectacles 5) there may be connected a power source and/or a data interface 9 by means of a cable (USB, for example) or by means of other communication means, including optical transmission or wireless transmission, such as via radio (Bluetooth or WiFi, for example). Through this data interface 9 the various parameters of the algorithms comprised in the processing means 6 may be configured, as indicated by the arrow from the data interface 9 to the processing means 6. Alternatively or additionally, the frame of the spectacles 5 may have integrated within it a battery 8 and the corresponding control electronics. The data interface 9 and/or the battery 8 may be coupled to the temple of the spectacles, or they may be situated in an auxiliary item such as a case, with a wired or wireless connection.

The system is designed to capture any object in a manner similar to the way in which the human eye focuses, that is, the system (specifically the camera 1) is able to focus automatically on any object situated between a minimum distance of about 15 cm from the camera 1 and a maximum distance of about 6 m from the camera 1. It is also possible to select the focus automatically at any time. This is achieved by optimising the system of lenses in the camera 1. The aforementioned focusing system 11 (a lens with the ability to change focus by means of a focusing motor in order to permit close or distant focusing, with an angle of view) helps to achieve optimal focus.

Thus, when the user with a macular disorder is wearing the spectacles 5 incorporating the system of the invention and looks at an object 12 situated in his or her field of view, he or she can see that object magnified (in general, modified) 32 on the display 3 in the following manner: The object is recorded by the camera 1, which sends the captured images or video to the processing means 6, which process them and supply them 22 to the projector 2, so that the latter can project on the screen or display 3 the processed image or video 32 for the user with a macular disorder to view. The images projected 32 on the screen or display 3 are magnified/improved/retouched virtual images of the captured object 12 that are more suitable for being interpreted correctly by the user (only one of whose eyes 13 is represented in figure 3). The treatment or processing that the images captured 12 by the camera 1 undergo until they are transformed into magnified/improved/retouched virtual images 22 that are subsequently projected 32 is described next. As has been mentioned, this treatment or processing takes place in the processing means 6. The processing means 6 comprise an ensemble/assembly of real-time video processing algorithms for the processing/improvement/retouching of images. These algorithms are implemented by means of software housed in said processing means 6 (in a memory associated therewith or belonging thereto, for example). Said software is executed in the processing means 6 so that the user can view in real time on the screen 3 the modified images of the object in front of him or her. With the sheet of variable opacity 4, the system can modulate the opacity of said sheet, situated superimposed or practically superimposed on the lens of the spectacles, in accordance with the ambient light and thereby improve the contrast of the image captured by the eye (thanks to the substantially opaque screen that is activated behind the screen or waveguide system). Both the display 3 and the additional sheet 4 are coupled to the lens of the spectacles or are situated in very close proximity thereto, in front of the eye affected by a macular lesion. The ensemble/assembly of processing algorithms is formed by the following algorithms:
(a) Magnification/reduction algorithm: this concerns a virtual optical enlargement so as to produce an effect of an enlargement/reduction xN, where N is a real number that varies preferably from 0.1 to 10, most preferably from 2 to 4. In figure 3, by way of example, the magnification is indicated to be x2. The magnification is produced starting from the optical centre of the captured image 13. Once the patient has been diagnosed and the size of the patient's macular lesion has been identified, the degree of magnification required by the patient may be selected. The magnification parameter may be changed, if necessary, by configuring the software embedded in the system of the invention, whenever the patient undergoes an ophthalmological check-up to determine the progress of his or her macular disease. That is to say, if in a check-up the macular lesion is found to have increased in size, the software may be configured so that the parameter N of the magnification algorithm is greater than in the configuration prior to the check-up. A non-limitative example of the implementation of these algorithms may be as follows:
   First, apply a zoom or enlargement of the image according to a variable value configured by the doctor. This value has no upper limit, it may be set with a precision of one decimal place, and it must be positive. Then, cut out the central area of the enlarged image, thereby obtaining an image at the same resolution as the original image (in one possible implementation, 640 * 480 pixels) but with a smaller field of view and with the objects enlarged. This process is equivalent to increasing the focal length of the lens of the camera 1 integrated on the spectacles 5.
(b) Filtering and improvement or chromatic compensation algorithms. These algorithms are optional. They concern the application of therapeutic chromatic filters to enhance certain colours, such as red or yellow, so as to favour the presence of wavelengths in the range of these colours and produce a clearer image. Therapeutic chromatic filters absorb short-wave radiation below 600 nm, preferably below 550 nm and most preferably below 520 nm (ultraviolet at approximately 400 nm, blue at approximately 450 nm and part of the green which lies approximately between 500 and 550 nm), resulting in an increase in yellow light (between approximately 555 and 600 nm) and red light (at approximately 700 nm). Since the photoreceptors (cones) of the retina are more sensitive to the wavelength of approximately 555 nm, the illumination of the retina is thereby accentuated and its sensitivity to contrast is increased. Moreover, these filters do not allow short-wave radiation which is harmful to the retina, such as blue and ultraviolet, to pass through. In this way, therefore, dazzle and chromatic aberration are reduced and contrast is notably increased.
   These filters may be customised depending on the patient's disorder and on whether the patient is in an indoor or outdoor environment. That is to say, a different filter, defined by its chromatic compensation vector, may be applied to each individual patient. For example:
   - a patient suffering from AMD should preferably use a filter of approximately 460 nm (a filter that does not allow wavelengths shorter than 460 nm to pass through) indoors and a filter of approximately 527 nm (a filter that does not allow wavelengths shorter than 527 nm to pass through) outdoors.
   - a patient suffering from diabetic retinopathy should preferably use a filter of approximately 527 nm (a filter that does not allow wavelengths shorter than 527 nm to pass through) indoors and a filter of approximately 540 nm (a filter that does not allow wavelengths shorter than 540 nm to pass through) outdoors.
   - a patient suffering from cone dystrophy should preferably use a filter of approximately 540 nm (a filter that does not allow wavelengths shorter than 540 nm to pass through) indoors and a filter of approximately 600 nm (a filter that does not allow wavelengths shorter than 600 nm to pass through) outdoors.
   A non-limitative example of an implementation of these algorithms follows:
   The colour blue is filtered in the RGB components so as to obtain the effect of intensifying the colour yellow in the image. The quantity of blue is filtered by using a multiplicative factor ranging between 0 and 1. The value 0 indicates total suppression of the blue component in the image, therefore enhancing the yellow component to the greatest degree possible. A value of 1 keeps the blue intact and the colour of the image undergoes no change at all.
   In these cases, the system comprises means to detect whether the user is indoors or outdoors in order to apply the appropriate filters for the situation in real time. For example, the system may detect whether the user is indoors or outdoors from the measurement of light intensity received by the microcamera 1.
(c) Algorithms to apply algorithms (a) and (b) in combination. As in the case of the chromatic compensation algorithms, this combination is optional. That is to say, a magnification (algorithms (a)) and a chromatic compensation (algorithms (b)) may be applied to one and the same image 12 recorded by the camera 1. The two algorithms are applied sequentially but produce a single combined output (result) of the two algorithms.
(d) Algorithms for the compensation of refractive errors, such as hypermetropia, astigmatism or myopia. These are also optional. These algorithms are applied in accordance with the vision disorders from which the user of the system suffers. These algorithms are preferably light deconvolution algorithms. A defocus opposite to that suffered by the person (who may be myopic, for example) is applied to the image from the camera so that, due to the defocus caused by the disorder (in this case myopia), the resulting image in the eye is focused. Once a patient with a macular disorder decides to use the system of the invention, the algorithms are configured so as to adjust them to the most suitable or optimised treatment for each individual patient. Examples of parameters subject to configuration are the parameter'N' of the magnification algorithm, the chromatic compensation vector (for chromatic filtering), and the number of dioptres for the diffraction error, among others.

With the system described herein, the patient with a macular disorder sees a considerable improvement in his or her central vision (the vision affected by the disease). Moreover, since the system is implemented in spectacles that have a 'see-through' display, the patient does not lose any peripheral vision, which is not affected by the system.

Furthermore, the invention is not limited to the specific embodiments described, but also covers, for example, variants that may be produced by the average person skilled in the art (for example, with regard to the selection of materials, dimensions, components, configuration, etc.), within the context that emerges from the claims.

## Claims

1. A system of assistance for a person with a macular disorder in at least one eye, wherein the system is integrated in or coupled to a pair of spectacles (5), **characterised in that** it comprises:
- a camera (1) configured to capture at least one image (12) situated in the field of view of said person and to measure the amount of ambient light;
- processing means (6) configured to process said image (13) captured by the camera (1), wherein said processing means (6) comprise software means for implementing at least one image-processing algorithm configured to obtain a virtual image retouched (22) in accordance with said macular disorder from said image (13) captured by the camera (1);
- a display (3) situated in front of the eye affected by a macular disorder;
- a projection system (2) configured to receive said virtual image retouched (22) in accordance with said macular disorder by the processing means (6) and to project (32) the retouched image (22) onto said display (3), so that said person with said macular disorder sees said retouched image projected (32) instead of the real image (12) present in the person's field of view; and
- a sheet of variable opacity (4) disposed next to the display (3), the display (3) being situated between the sheet of variable opacity (4) and the person's eye (13), the sheet of variable opacity (4) being configured to modulate its opacity in accordance with the ambient light and thus improve the contrast of the image projected (32) on the display (3).

2. The system according to claim 1, wherein said processing means (6) are embedded in the frame of a pair of spectacles (5).

3. The system according to either of the preceding claims, wherein said camera (1) is situated on the front part of the frame of a pair of spectacles (5), or in the area in which the temple begins, or on the bridge intended to rest over the nose.

4. The system according to any of the preceding claims, wherein said sheet of variable opacity (4) is either an electrochromic crystal, which changes its opacity through an electric voltage applied in accordance with the ambient light level detected by the camera (1), or a photochromic crystal, which changes its opacity in accordance with the ambient light directly.

5. The system according to any of the preceding claims, wherein said projection system (2) comprises a plurality of light sources and diffraction means.

6. The system according to any of the preceding claims, wherein said display (3) comprises a waveguide system.

7. The system according to any of the preceding claims, wherein said at least one image-processing algorithm configured so as to obtain a retouched virtual image (22) comprises a magnification algorithm configured so as to enlarge/reduce the captured image (13) N times, N being a real number varying between 0.1 and 10, most preferably between 2 and 4.

8. The system according to claim 7, wherein said at least one image-processing algorithm configured to obtain a retouched virtual image (22) comprises at least one filtering and chromatic compensation algorithm configured to absorb short-wave radiation below 600 nm.

9. The system according to either claim 7 or claim 8, wherein said at least one image-processing algorithm configured to obtain a retouched virtual image (22) comprises at least one algorithm for the compensation of refractive errors.

10. An ensemble for the assistance of a person with a macular disorder in at least one eye, comprising:
- a spectacle frame (5); and
- a system according to any of claims 1 to 9, installed on said spectacle frame (5).

11. A method of assistance for a person with a macular disorder in at least one eye, comprising:
- capturing in a camera (1) an image (13) situated in the field of view (12) of said person, and measuring the amount of ambient light;
- processing (6) said image (13) captured by the camera (1) to obtain a retouched virtual image (22) of the captured image (13);
- projecting (32) said processed image (22) from a projector (2) onto a display (3) situated in front of the eye affected with said macular disorder;
- improving the contrast of the projected image (32) by means of a sheet of variable opacity (4) disposed next to the display (3).

12. The method according to claim 11, wherein said stage of processing (6) said captured image (12) comprises applying at least one image-processing algorithm configured to obtain a retouched virtual image (22) from said image (12) captured by the camera (1 ).

13. The method according to claim 12, wherein said at least one algorithm comprises enlarging/reducing the image, modifying its size N times, N being a real number varying between 0.1 and 10, most preferably between 2 and 4.

14. The method according to any one of claims 11 to 13, wherein said at least one algorithm comprises filtering the image, applying chromatic compensation to it, based on absorbing short-wave radiation below 600 nm, and optionally compensating for refractive errors.

15. A computer program that comprises computer programming code instructions for implementing the method of any one of claims 11 to 14.
